# EUROPEAN PATENT SPECIFICATION

(11) **EP 1 878 455 B1**
(45) Date of publication and mention of the grant of the patent: **02.06.2010**
(21) Application number: 07111632.1
(22) Date of filing: 03.07.2007
(51) Int. Cl.: A61M 15/00, A61M 5/168

(54) **Method for controlling ejection of medicines and medicine ejection apparatus**
Verfahren zur Steuerung des Ausstoßes von Medikamenten und Vorrichtung zum Ausstoß von Medikamenten
Procédé de contrôle d'éjection de médicaments et appareil d'éjection de médication

(30) Priority: 13.07.2006 JP 2006192905; 15.06.2007 JP 2007158603
(43) Date of publication of application: 16.01.2008
(73) Proprietor: CANON KABUSHIKI KAISHA, Ohta-ku Tokyo 146-8501 (JP)
(72) Inventor: Sugita, Masaru, Tokyo Tokyo 146-8501 (JP); Hamano, Soji, Tokyo Tokyo 146-8501 (JP); Watanabe, Shinji, Tokyo Tokyo 146-8501 (JP); Imai, Mitsuru, Tokyo Tokyo 146-8501 (JP)
(74) Representative: TBK-Patent

(56) References cited:
- EP-A- 1 110 567
- US-A- 5 002 048
- US-A- 5 522 798
- US-B2- 6 684 880

## Description

### BACKGROUND OF THE INVENTION

### Field of the Invention

The present invention relates to a method for controlling an apparatus for the ejection of a plurality of medicines, and to a medicine ejection apparatus capable of ejecting a plurality of medicines.

### Description of the Related Art

An ink jet technique is widely known as a method for ejecting liquid. For example, in a thermal jet method, a liquid in an ejection energy working section communicating with an ejection port is heated by a heat resistor to generate bubbles, and the bubbles act to eject the liquid through the ejection port. In a piezoelectric method, a piezoelectric element applies mechanical energy to eject the liquid. The ink jet technique is advantageous in that the size and amount of ejected droplets can be precisely controlled even if they are small. Accordingly, it is expected that the ink jet technique will be applied to, for example, an inhalation apparatus allowing transpulmonary administration of pharmaceutical liquid (see U. S. Patent No. 5,894,841).

Typical medical inhalers include suspension aerosol type metered dose inhalers (MDI), dry powder inhalers (DPI), and nebulizers.

Some of the medicine ejection apparatuses eject not only a single composition, but also a plurality of substances. For example, a variety of combinations are sprayed, such as a pharmaceutical compound and an adjuvant or a plurality of pharmaceutical compositions. Such pharmaceutical compounds include therapeutic compounds, perfumes, and coloring agents and are used for a wide range of applications.

U. S. Patent No. 6,684,880 has disclosed an ejection apparatus ejecting a plurality of medicines by an ink jet technique, and which includes a plurality of medicine reservoirs and a plurality of ejection heads corresponding to the respective reservoirs. The medicine reservoirs hold the medicines, and the medicines may be ejected from the respective ejection heads simultaneously or one after another.

If a plurality of medicines are inhaled in an inappropriate order unfortunately, intended efficacy may not be produced. For example, a diabetic suffering from asthma or bronchitis should inhale a bronchodilator first with an inhaler to expand the bronchus and then inhale insulin to increase the deposition of insulin in the lung. If insulin is inhaled without first inhaling the bronchodilator, a large proportion of the insulin droplets are trapped in the bronchus and will not reach the alveolus. Insulin is effective when it is absorbed from the alveolus to the capillaries to be carried by the bloodstream. However, if the insulin is trapped in the bronchus, it is absorbed slowly by the blood vessel, and part of it may not be absorbed by the blood vessel. This is disadvantageous for diabetics, because the actual dose absorbed is reduced in spite of the importance of ingesting a predetermined amount of insulin every time.

However, the above-cited U. S. Patent No. 6,684,880 has disclosed only that a plurality of medicines are ejected one after another, but has not disclosed the order or timing for ejecting different types of medicines. The ejection apparatus of U. S. Patent No. 6,684,880 is not designed to eject a plurality of types of medicines in an appropriate order. Hence, the user of the apparatus needs to consider the appropriate inhalation order, and if the user fails to consider such order, inhalation may not be made in the appropriate order.

US-A-5 522 798 discloses a method for controlling a multichannel drug infusion pump using a pharmacokinetic model. According to the disclosure of this document, a medicine injection apparatus for ejecting a plurality of medicines in a plurality of reservoirs is provided, wherein the apparatus comprises a section for the identification of the medicines (a bar code reader), a section which sets the ejection order of the medicines (a host controller) and a section which actually ejects the medicines in the order set in the previous section (the drug channels).

### SUMMARY OF THE INVENTION

The present invention provides a method for controlling an apparatus for the ejection of medicines so that the user can simply and easily inhale a plurality of medicines from a single ejection apparatus in an appropriate order without particular consideration, and a medicine ejection apparatus embodying the method.

The present invention in its first aspect provides a method for controlling an apparatus for the ejection of at least two medicines as specified in claims 1 to 11.

The present invention in its second aspect provides a medicine ejection apparatus as specified in claims 12 to 14.

Other features and advantages of the present invention will be apparent from the following description taken in conjunction with the accompanying drawings, in which like reference characters designate the same or similar parts throughout the figures thereof.

### BRIEF DESCRIPTION OF THE DRAWINGS

Fig. 1 is a block diagram of a principal structure of a medicine ejection apparatus according to an embodiment of the present invention.

Fig. 2 is a schematic diagram of a cartridge of a medicine ejection apparatus according to an embodiment of the present invention.

Fig. 3 is a perspective view of an inhaler using a medicine ejection apparatus according to an embodiment of the present invention.

Fig. 4 is a perspective view of the inhaler shown in Fig. 3 when the access cover is open.

Fig. 5 is a block diagram of a medicine ejection apparatus according to an embodiment of the present invention.

Fig. 6 is a flow diagram through which the medicine ejection apparatus shown in Fig. 5 decides an order in which medicines are ejected.

Fig. 7 is a block diagram of a medicine ejection apparatus according to another embodiment of the present invention.

Fig. 8 is a block diagram of a medicine ejection apparatus according to still another embodiment of the present invention.

Fig. 9 is a flow diagram through which the medicine ejection apparatus shown in Fig. 7 or 8 decides an order in which the medicines are ejected.

Fig. 10 is a schematic diagram of a nebulizer-type or powder discharge-type cartridge using a piezoelectric element.

### DESCRIPTION OF THE EMBODIMENTS

Preferred embodiments of the present invention will now be described in detail in accordance with the accompanying drawings.

"Ink jet" mentioned herein broadly refers to ejecting not only a printing ink, but also other liquid, such as medical liquid. "Ink jet" may be referred to as "liquid jet".

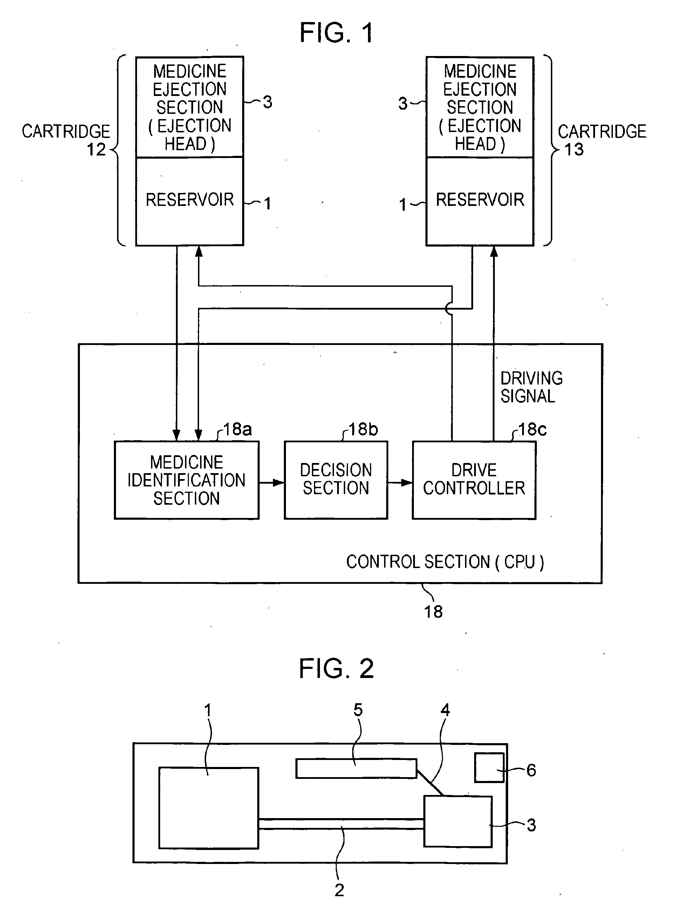

Fig. 1 is a block diagram of a principal structure of a medicine ejection apparatus according to an embodiment of the present invention. In the following description, a cartridge-type medicine ejection apparatus which includes a cartridge will be described. The cartridge includes an integrated set of a reservoir 1 holding a medicine and an ejection head 3. The medicine ejection apparatus can have a plurality of cartridges, and the medicine ejection apparatus shown in Fig. 1 has two cartridges 12 and 13. The reservoir may be referred to as a container. The medicine ejection apparatus includes a body containing a control section (CPU) 18. The control section includes a medicine identification section 18a that identifies the medicines contained in the reservoirs in the cartridges, a decision section 18b that decides the ejection order in which the medicines are ejected according to the combination of the medicines identified by the medicine identification section 18a, and a drive controller 18c that controls the operation of the ejection heads ejecting the medicines. The drive controller 18c sends a signal to control the ejection heads so as to eject the medicines from the reservoirs according to the order decided by the decision section 18b. Thus, the medicine ejection sections 3 of the cartridges 12 and 13 start ejecting a plurality of medicines according to the ejection order decided by the decision section 18b.

A method for controlling an apparatus for the ejection of medicines according to an embodiment decides an ejection order in which at least two types of medicines are ejected according to the combination of the medicines, and ejects the medicines one after another in the decided ejection order. When several types of medicines are inhaled, the medicines should be inhaled in an appropriate order. For example, as described above, when using a combination of a bronchodilator and insulin, it is desirable that the bronchodilator be inhaled first, and then the insulin. If a specific combination is determined for a method for controlling an apparatus for the ejection of medicines to accomplish such an appropriate inhalation, an ejection order for the combination is decided and the medicine ejection apparatus operates so as to eject the medicines in the decided order. The medicine ejection apparatus embodying this method is configured to decide an appropriate ejection order according to the combination of a plurality of medicines in reservoirs and to control an ejection mechanism to eject the medicines in the ejection order.

The medicine ejection apparatus of the invention may use a plurality of cartridges, each including a reservoir. The cartridge may include an integrated set of an ejection head and the reservoir. The ejection head may be provided in the medicine ejection apparatus separately from the reservoir. If the ejection head is directly provided in the medicine ejection apparatus, a plurality of ejection heads may be disposed corresponding to the respective cartridges, or a single head may be used. In the following description, the cartridge includes a reservoir regardless of whether the reservoir and the ejection head are integrated or separate. The number of cartridges can be arbitrarily set according to, for example, the types of medicines inhaled at one time. The ejection order can take a variety of patterns according to the combination of medicines.

For example, in a structure having three cartridges respectively including reservoir A containing medicine a, reservoir B containing medicine b, and reservoir C containing medicine c, medicines a, b, and c may be ejected in that order, or a specific medicine may be ejected several times in an order of, for example, medicine a, medicine b, medicine a, and medicine c. An ejection sequence may be repeated in an order of, for example, a, b, c, a, b, and c. Furthermore, a first medicine or a second medicine may be ejected as a group of a plurality of medicines. More specifically, medicine a may be ejected as the first medicine, and then medicines b and c may be simultaneously ejected as the second medicine. The ejection order and the number of ejection times of medicines thus can be arbitrarily set according to the types of medicines.

The second medicine may be ejected after the ejection of the first medicine has been stopped, or it may be ejected before the ejection of the first medicine is stopped. In the former case, each medicine is ejected separately and a single medicine is ejected at a time. In the latter case, the ejections of the first and second medicines are not started or completed at exactly the same time, but the ejection of the two medicines can overlap for a period of time.

The sequence of ejection is appropriately set according to the combination of medicines to be ejected.

The medicine mentioned herein refers to a medical compound having a pharmacological or physiological function, but also a taste or flavor component, or a dye or pigment. The medicine may be liquid or powder.

The medicine liquid mentioned herein refers to a liquid medicine or a liquid medium containing a pharmaceutical compound. The pharmaceutical compound in the liquid may be dissolved, dispersed, emulsified, or suspended, and is preferably homogenized in the liquid.

When a medicine liquid is used as the medicine, the main medium of the liquid can be water or an organic material, and is preferably water from the viewpoint of administration to a living body.

The above-mentioned medical compound having a physiological function may be a generally used pharmaceutical compound. Examples of such pharmaceutical compounds include anti-inflammatory steroids, non-steroidal anti-inflammatory agents, sedatives, therapeutic agents against melancholia, analgesics, antasthmatics, β-sympathetic agents, anticholinergic agents, mast cell stabilizers, and antagonists. In addition, the pharmaceutical compounds include anti-tussive, expectorants, antihistamines, antiallergic agents, antiemetics, somnifacients, vitamin compounds, sex steroid hormone, anti-tumor agents, anti-arrhythmic agents, hyper-tensive agent, anti-anxiety agents, anti-psychotic agents, cardiac stimulants, and bronchodilators. Furthermore, the pharmaceutical compounds include bariatric medicines, migraine medicines, anti-rheumatics, protein preparations, hormones, cytokine, receptors, antibodies, enzymes, enzyme inhibitors, vaccines, viruses, antisense strands, genes, and nucleic acids.

Although the amount of medicine varies with each substance, it can be set in the range of 1 ppm by weight to 10% by weight, and preferably in the range of 0.001% to 5% by weight.

Taste or flavor components used herein include natural perfumes, synthetic perfumes, and compound perfumes. General perfume components may also be used, such as perfumes used in cosmetics, soaps, and food. It is preferable to use medical perfumes pharmacopeially defined as subcomponents or perfumes permitted to be added to food or cosmetics.

Although the content of perfume or the like added as a taste or flavor component varies with each type of perfume, it is generally set in the range of 1 ppb by weight to 10% by weight, and preferably in the range of 1 ppm by weight to 1% by weight. A taste component and a flavor component may be used in combination within the intended purpose of the ejection liquid.

A variety of dyes and pigments can be used as a coloring agent, and it is preferable to use medical substances pharmacopeially defined as subcomponents or substances permitted to be added to food or cosmetics.

Although the content of the coloring agent, such as dye or pigment, depends on the type of the coloring agent to be used, it is generally set in the range of 1 ppm by weight to 30% by weight, and preferably in the range of 0.01% to 10% by weight. A dye and a pigment may be used in combination within the intended purpose of the ejection liquid.

An additive, such as an ejecting adjuvant or an absorption promoter, may be added if necessary. The pharmaceutical compound, perfume, or coloring agent may be a hydrophobic material not exhibiting desired solubility. In this instance, a dispersant, a surfactant, or the like may be added so that the hydrophobic material can be uniformly dispersed. In addition, additives appropriate to the intended purpose of the ejection liquid may be added in appropriate proportions, such as a dispersant, a surfactant, a surface conditioner, a viscosity modifier, a solvent, a moisturizing agent, and a pH adjuster.

Examples of additives used herein include ionic surfactants, nonionic surfactants, emulsifiers, dispersants, hydrophilic binders, hydrophobic binders, hydrophilic thickeners, hydrophobic thickeners, glycerol, glycols, and glycol derivatives. In addition, the additives include alcohols, amino acids, urea, electrolytes, and buffer components. The above-listed additives may be used singly or in combination as required.

In use of such additives, it is preferable to use medical substances pharmacopeially defined as subcomponents or substances permitted to be added to food or cosmetics.

The additive content (on a mass basis) depends on the types and contents of the pharmaceutical compound being the principal component, the perfume used as the taste or flavor component, and the coloring agent, and can be defined as below. In general, the additive content is preferably in the range of 0.01% to 40% by weight, and more preferably 0.1% to 20% by weight. The amount of additive may be arbitrarily set depending on the function, type and combination. Preferably, the amount of additive is in the range of 0.5 to 100 parts by mass relative to 1 part by mass in total of the pharmaceutical compound, the flavor or taste component, and the coloring agent.

Liquid compositions filling the plurality of reservoirs are prepared as above. The liquids in the reservoirs may be the same or different, but preferably different. More specifically, the liquids may be pharmaceutical compounds, or a combination of a pharmaceutical compound and a surfactant. The liquid composition in each reservoir may be a mixture of a pharmaceutical compound, a perfume, or a coloring agent and an additive, or a mixture of substances selected from among pharmaceutical compounds, perfumes, and coloring agents.

The time interval (in a cycle) between the time at which the ejection of the first medicine is started and the time at which the ejection of the second medicine is started may arbitrarily be set. Preferably, the second medicine is ejected after the first medicine produces its in vivo efficacy. More preferably, the second medicine is ejected when the first medicine produces higher in vivo efficacy. The time intervals may be the same or vary with each cycle.

If the second medicine is ejected after the ejection of the first medicine has been stopped, the time interval between the time at which the ejection of the first medicine is stopped and the time at which the ejection of the second medicine is started may also arbitrarily be set, but preferably in the range of 1 second to 10 minutes. By setting this time interval to 1 second or more, the user is not required to inhale the two medicines during only one breath, but can inhale the second medicine with another breath after inhaling the first medicine and subsequently taking a breath. However, a time interval of 10 minutes or more after the inhalation of the first medicine may weaken the efficacy of the first medicine, or cause the user to forget to inhale the second medicine.

The method for controlling an apparatus for the ejection of medicines of the present invention can be suitably applied to cases where the first medicine enhances the in vivo efficacy of the second medicine. Exemplary combinations capable of enhancing in vivo efficacies will be described below.

For example, if a bronchodilator is used as the first medicine, the second medicine can be inhaled after the bronchus is sufficiently expanded, so that the second medicine can readily reach a desired deposition site. If a medicine intended for transpulmonary absorption or treatment of deep lung diseases is used as the second medicine, the deposition of such a medicine in the lung can be increased.

Therefore, users who simultaneously suffer from asthma and diabetes can inhale a bronchodilator and then a medicine containing insulin or GLP-1. Exemplary medicines intended for transpulmonary absorption include growth hormones, interferon, and cytokine. "Insulin" mentioned herein broadly refers to not only a normal insulin, but also insulin analogue and insulin derivative. Examples of insulins include insulin, products thereof with modified amino acid sequences such as insulin aspart, insulin lispro, insulin glargine and insulin detemir. In addition, any peptide portion of insulins mentioned above, which has the whole or part of the main structure of the above substance and at least part of biological characteristics of insulin, can be also used. "GLP-1" mentioned herein broadly refers to not only a normal GLP-1, but also GLP-1 analogue.

Exemplary medicines for treating deep lung diseases include antibiotics, steroid, anticholinergic agents, and B2 stimulants.

A combination of a bioactive substance and an inhibitor of an enzyme capable of decomposing or metabolizing the bioactive substance is also effective.
For example, DPP-4 inhibitor is an inhibitor of an enzyme capable of decomposing GLP-1 promoting in vivo insulin secretion. Hence, it is effective when a medicine liquid containing DPP-4 inhibitor and then a medicine liquid containing GLP-1 are ejected in that order. Examples of DPP-4 inhibitor include sitagliptin, vildagliptin, and so on.

It is also effective when the second medicine has the efficacy of suppressing side effects of the first medicine. For example, if an inflammation is produced after the first medicine is inhaled, an anti-inflammatory is used as the second medicine to suppress the inflammation. In addition, an absorption promoter may be combined with a remedy to enhance the absorption. A combination of a remedy and a perfume allows the ejection of medicines to be confirmed. A combination of a plurality of perfumes and aromatherapy medicines can produce a variety of efficacies or a new efficacy.

In addition to transpulmonary absorption, the ejection control method of the invention can be applied to delivery of medicines through mucous membranes to eyes, the nasal cavity and the oral cavity as with the lung.

Three medicines may be ejected. For example, a bronchodilator and a DPP-4 inhibitor are ejected and then GLP-1 is inhaled.

When the ejection of the second medicine is started after the ejection of the first medicine is stopped, it may be preferable that the time interval between the time at which the ejection of the first medicine is stopped and the time at which the ejection of the second medicine is started is in the range of 0.00001 second to a period for one inhalation. In this instance, medicine liquids contained in the respective reservoirs are switched every millisecond and ejected separately. By repeating this sequence, different types of medicine liquids can be repeatedly ejected separately every millisecond.

This method can facilitate the ejection of, for example, a combination of a remedy, a perfume and an analgesic, and alleviate the discomfort or distaste felt when the remedy is inhaled. Thus, the user can inhale the medicines comfortably. Only the perfume and the analgesic may be inhaled at the beginning of aspiration, and then the perfume, the analgesic and the remedy may be repeatedly ejected one after another every millisecond. Thus, the user can inhale the medicines comfortably without experiencing the bitterness of the remedy.

The timing of start and stop of ejections can be controlled by electronic control with a program such as computer-executable program stored on a computer readable medium for controlling the operation of the ejection sections. Thus, the amounts of medicine liquids to be ejected and the ejection timing can be precisely controlled, and accordingly the ejection can be performed with high repeatability, accuracy and consistency.

The electronic control can be performed by a vibratory technique such as using a piezoelectric actuator or ultrasonic waves, or a negative pressure technique such as liquid bubbling by applying thermal energy. The liquids may be delivered by any technique in the invention.

Since the method of the present invention allows a plurality of medicines to be handled separate from one another, it is not necessary to take into account the stability in storage or preservation (so called pot life).

Although in the above embodiment, the plurality of reservoirs hold different types of medicines, they may hold the same medicine to increase the ejection quantity.

The medicines can be ejected by applying thermal energy to the medicines using an electro-thermal conversion element, or applying mechanical energy to the medicines using vibration pressure of an electromechanical conversion element (for example, piezoelectric element). The ejection method may be selected according to the types of medicines.

In the field of printers, the technique of applying thermal energy with an electro-thermal conversion element is referred to as a "thermal ink jet method" and the technique of applying mechanical energy with an electromechanical conversion element is referred to as a "piezoelectric method", for the sake of convenience. These terms are also used for medicine ejection, but simply mean that energy for ejection is applied on the basis of the principle of the ink jet method.

The thermal ink jet method can increase the diameter of the ejection port, the amount of pulsed heat used for ejection, the dimensional precision of the micro-heater or the like used for the heat, and the repeatability, for each liquid ejection section. Consequently, ejection with a narrow distribution in droplet size can be achieved. In addition, the manufacturing cost of the head is reduced, and accordingly, the thermal ink jet method can be suitably applied to apparatuses having small heads that require frequent replacement. The thermal ink jet method is particularly suitable for use in a liquid ejection apparatus required to be portable and convenient.

Although the nozzle diameter of each ejection section may be arbitrary, it is preferable that the diameter be appropriately set according to the type of medicine to be ejected. For lung inhalation, the droplets of the preparation according to the embodiment must have diameters in the range of 0.5 to 20 µm and can be ejected with a narrow distribution.

Thus, the site in the lung that the spray of droplets reaches can be changed. In order to deliver the droplets to the alveolus, the diameter is preferably about 2 to 3 µm. For delivery to the bronchus or airway, the diameter is preferably about 7 to 8 µm.

Thus, the ejection control method allows a plurality of medicines to be ejected and inhaled, thereby helping the medicines produce their full efficacy.

A medicine ejection apparatus according to an embodiment will now be described with reference to the drawings. The medicine ejection apparatus includes a decision section that decides an order at which a plurality of medicines contained in respective reservoirs are ejected, according to the combination of the medicines, and a drive controller that controls the operation of a medicine ejection section so that the medicines are ejected from the reservoirs in the decided order.

Fig. 2 is a schematic diagram of a cartridge of the medicine ejection apparatus according to the present embodiment. The cartridge shown in Fig. 2 includes an ejection head 3 (medicine ejection section) for ejecting liquid, a reservoir 1 containing the liquid, and a liquid flow path 2 delivering the liquid from the reservoir 1 to the ejection head 3. The ejection head 3, the reservoir 1, and the liquid flow path are disposed together on a substrate. The ejection head 3 exchanges driving signals and control signals with a controller (drive controller) controlling the operation of ejection energy generating elements through an electrical connection 5 to which an internal wire 4 is connected.

In an embodiment of the invention, an identification code 6 is provided to the cartridge to identify the medicine in the cartridge. The identification code 6 of the cartridge may be a bar code, a QR code, an RF tag (for radio frequency identification, RFID), or an IC tag (for an IC chip), which are known as codes capable of identifying medicines. The identification code can be read by a known technique using, for example, images, electricity, or radio waves. Specifically, the code can be read with a CCD, a CMOS device, an electrical contact, or an antenna.

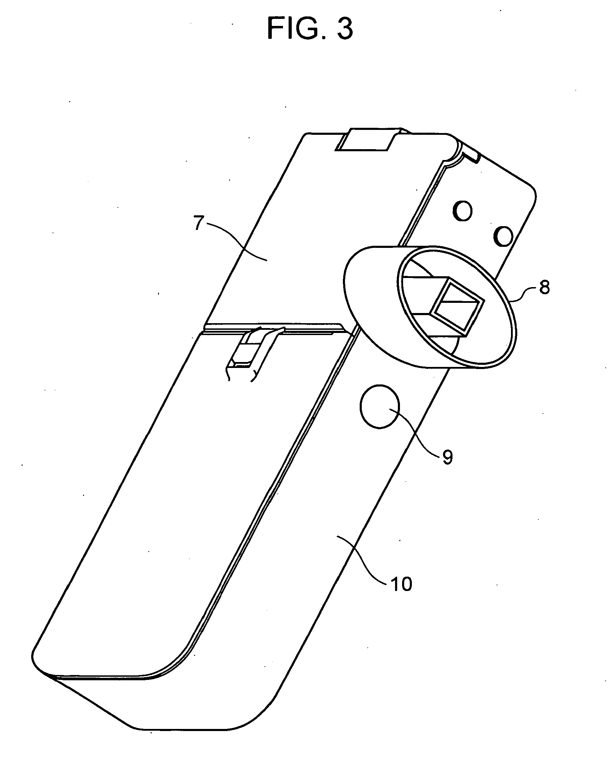

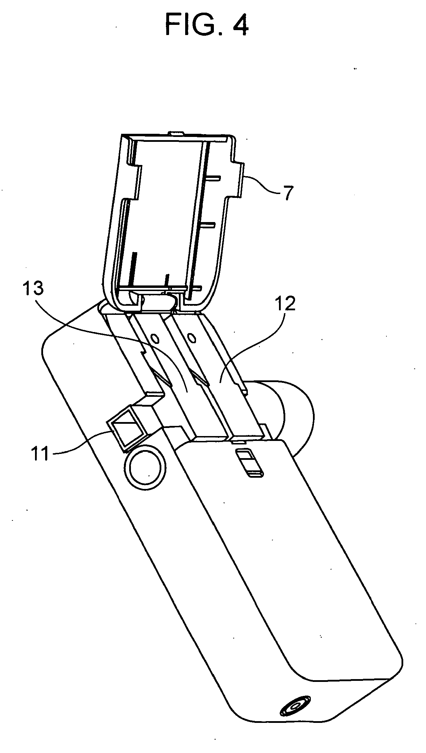

Figs. 3 and 4 show an inhaler being a type of the medicine ejection apparatus that is downsized so as to be easily carried by the user. Fig. 3 is a perspective view of the inhaler. The inhaler includes a body 10 containing medicine ejection cartridges, a controller of the cartridges, a power source (battery) and so forth, a mouthpiece 8 that is put in the mouth for inhalation, an access cover 7, and a power button 9. The medicine ejection cartridge has an integrated set of a reservoir and an ejection head as shown in Fig. 2, and can be replaced with the access cover 7 open. Fig. 4 is a perspective view of the inhaler when the access cover is open 7. The cartridges 12 and 13 are disposed in communication with an air tube conducting air to the airflow path from an air inlet 11. Medicines are sprayed as fine particles from the ejection heads of the cartridges 12 and 13 and mixed with each other in the airflow through the air tube. For use of the inhaler, the user breathes in with the mouthpiece 8 held in his or her mouth, and thus air comes through the air inlet.

The structure shown in Fig. 3 allows the fine spray of medicine droplets to naturally reach the throat and trachea of an inhalation subject together with aspirated air.

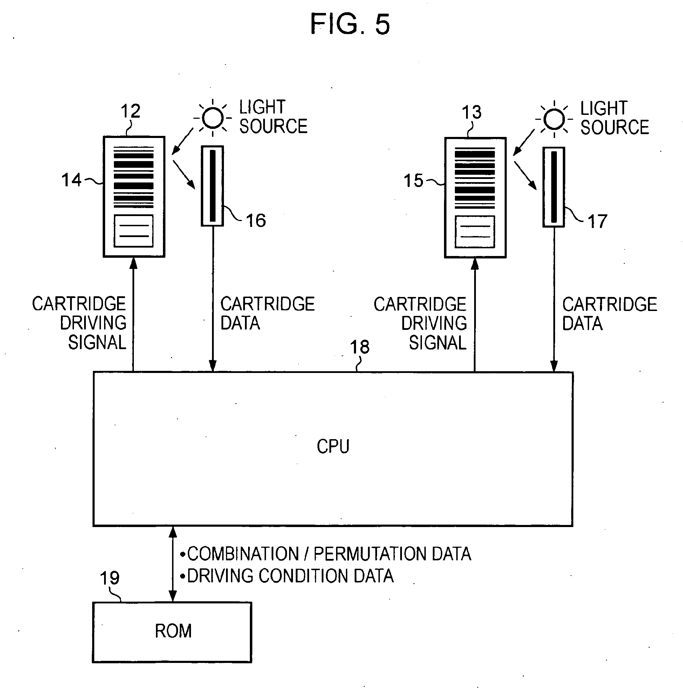

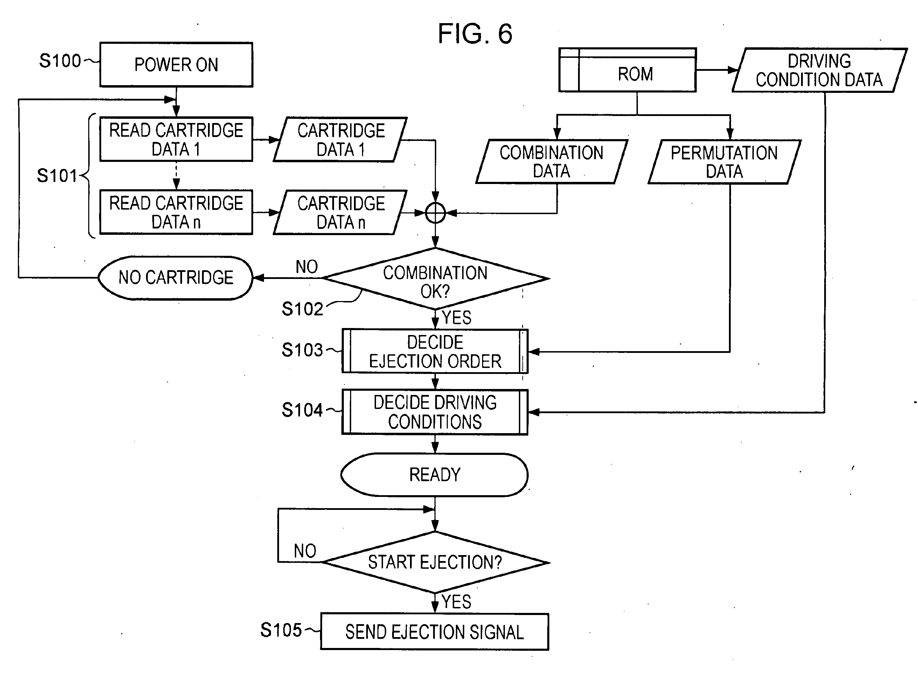

Fig. 5 shows a medicine ejection apparatus according to the embodiment of the invention. Fig. 5 omits the medicine identification section, decision section, and drive controller of the control section 18. Fig. 6 shows a flow diagram of a procedure for deciding an ejection order. A procedure for deciding an ejection order will now be described in detail referring to Figs. 5 and 6. First a user presses the power button 9, and the apparatus is powered on (S100). The cartridge identification codes 14 and 15 of the cartridges 12 and 13 are read with readers (CCD) 16 and 17 of the medicine ejection apparatus respectively (S101). The control section 18 identifies the medicines in the reservoirs of the cartridges on the basis of the cartridge data transmitted from the readers and determines the combination of the medicines (S102). When the cartridge is not set, the apparatus instructs to set the cartridge. The apparatus includes a memory section (ROM) 19 that stores information of appropriate ejection order for combinations of medicines in a table. The control section 18 collates the identified combination data with the information in the memory section 19 and decides the order in which the cartridges are operated, according to an appropriate order for the combination (S103). It is preferable for the driving condition to be set in the memory unit 19 beforehand by doctors, so the control section 18 decides driving conditions referring to the memory unit 19 (S104). The control section 18 sends a driving signal according to the decided operational order to each cartridge, and thus the cartridges are sequentially operated (S105).

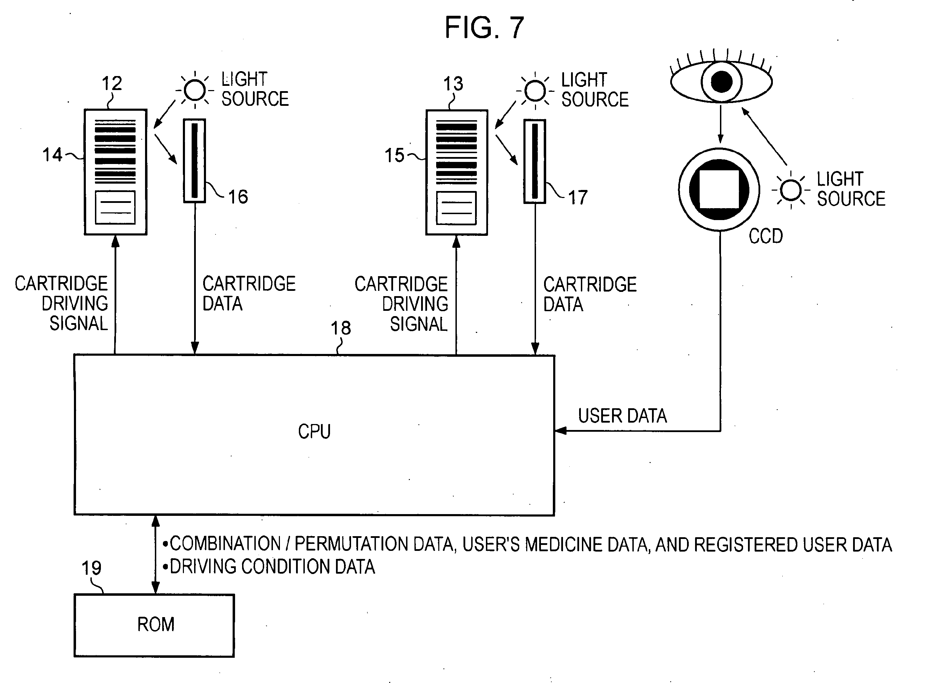

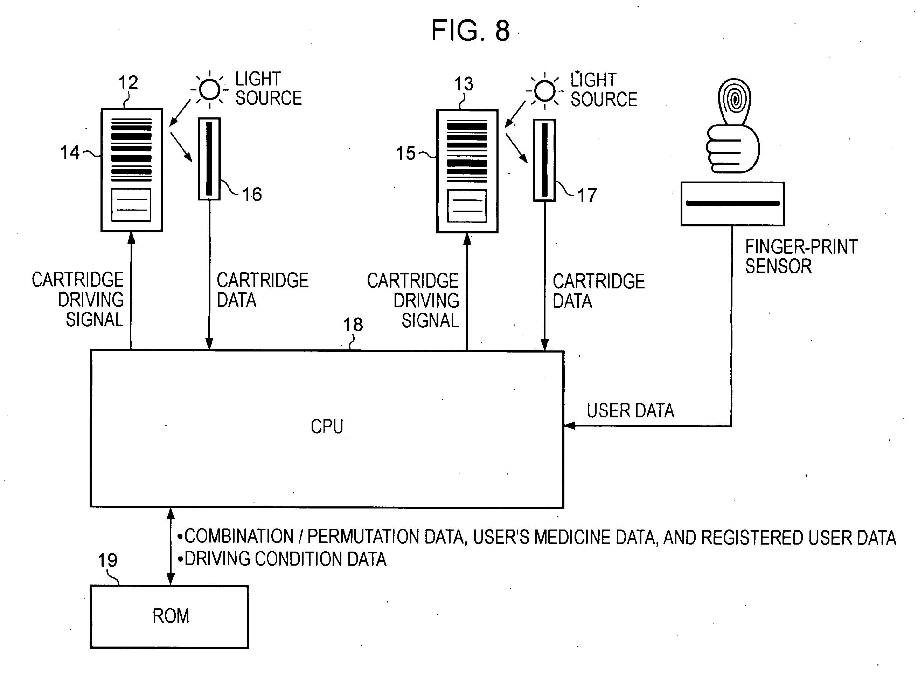

In another embodiment of the present invention, the medicine ejection apparatus may control the ejection of medicines according to the user data in addition to the cartridge identification data. In this embodiment, the medicines that the user uses are registered in the memory section 19 in advance. The memory section 19 stores the information of ejection orders for the combinations of the user's medicines and user information for user identification. On identifying a specific user by inputting user data, the medicine identification section 18a of the control section 18 knows that the registered medicines of the user should be ejected, and determines what medicines are contained in the respective cartridges by the above-described cartridge identification. The operational order of the cartridges is thus decided. A driving signal according to the operational order is sent to the cartridges and the cartridges operate in the decided order. Bio-information of users may be used as user information. For example, as shown in block diagrams Figs. 7 and 8, irises and fingerprints may be used for data collation. For example, the iris or fingerprint of a user is read with a reader, such as a CCD or a finger print sensor, and the user data is transmitted to the control section 18.

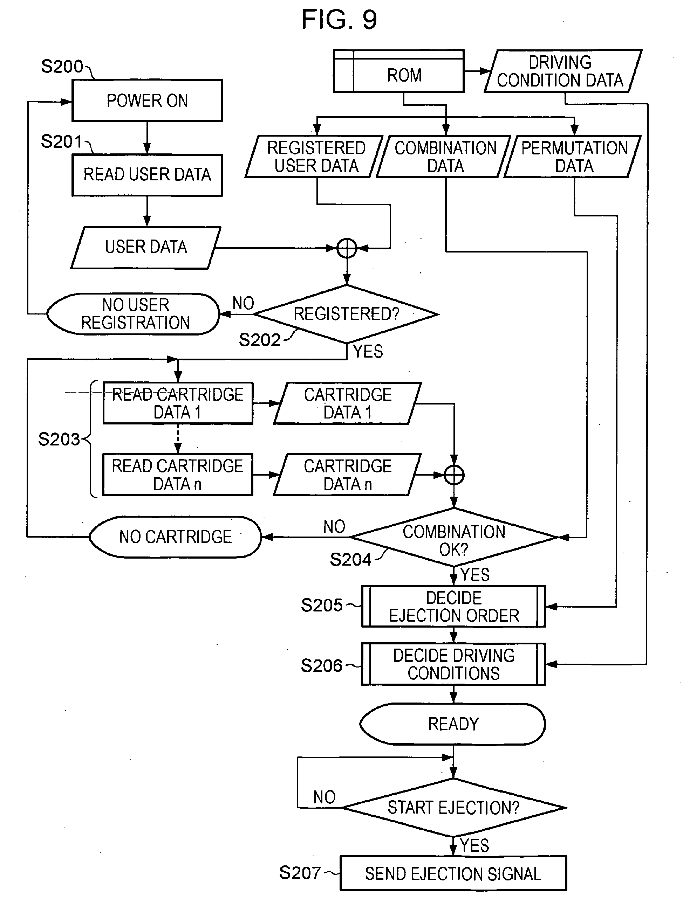

Fig. 9 shows the flow diagram of this procedure. After turning on the power (S200), user data is input. For example, the iris or fingerprint of a user is read with a reader, such as a CCD or a finger print sensor, and the user data is transmitted to the control section 18 (S201). The apparatus knows the types of medicines by the identification of the user (S202). Then, the cartridges are identified by the above-described procedure (S203) to determine what medicines are contained in the respective cartridges (S204). It is preferable for the driving condition to be set in the memory unit 19 beforehand by doctors, so the control section 18 decides driving conditions referring to the memory unit 19 (S205). Then, the ejection order is decided on the basis of the information in the memory section (ROM) 19 (S206) and a driving signal is transmitted to the cartridges. Thus, the cartridges are sequentially operated (S207).

If what medicines are to be placed in the cartridges is stored in the memory section 19 corresponding to the cartridges, the ejection order can be decided only by user identification without identifying the cartridges.

In still another embodiment of the present invention, the user may input what medicine is used. By inputting medicine data, the control section (medicine identification section 18a) of the apparatus acquires information as to what medicines are held in the reservoirs. Then, the data is collated with the information in the memory section 19 to decide the ejection order, and a driving signal is transmitted from the drive controller 19c to each cartridge so that the medicines are ejected in the decided order.

In addition, the ejection method may be stored in the memory section in advance, and appropriate ejection conditions may be selected by extracting necessary information according to the combination of the medicines. Alternatively, predetermined ejection conditions may be fixed.

If the user knows the information of ejection when the second medicine is ejected after the ejection of the first medicine is finished, the user can inhale the medicines without anxiety. Exemplary methods for this purpose include lighting with, for example, a diode, auditory signs with a buzzer, a sound, or music, vibration using a vibration motor, or display on a panel using characters or images. For example, for displaying information, the type of the second medicine, the time until the ejection is started, or the duration of ejection is desirably displayed.

The nozzle diameter (for example, ejection port diameter) of the medicine ejection section of the cartridge may be arbitrary selected, but can be appropriately set according to the type of the medicine ejected through the nozzle.

The operation of the apparatus is basically started by a single action. The action is defined as work until an administration is completed after the apparatus is set up.

The ejection of the first medicine may be started in synchronization with an inhalation of the user, or by pressing a button depending on the decision of the user. Since the apparatus is controlled so that the ejections are automatically switched according to the decided order after the ejection of the first medicine is started, the user can inhale a plurality of types of medicines by a simple action of, for example, starting inhalation or pressing a button.

Examples of the invention will now be described, but the invention is not limited to the examples.

### EXAMPLE 1

Two types of medicine liquids were ejected by the inhaler shown in Fig. 3. First, solutions were prepared in which medicines were to be dissolved or dispersed, as follows. Ejection liquids were prepared using the solutions selected depending on the medicines to be used.
Solution A: 2 mg/mL lauroylsarcosine aqueous solution
Solution B: 10 mg/mL arginine hydrochloride aqueous solution
Solution C: 10 mg/mL benzalkonium chloride aqueous solution

Salbutamol sulfate and insulin were selected as pharmaceutical compounds and were dissolved or dispersed in solutions A and B to prepare 0.5% and 0.4% ejection liquids respectively. The liquids were placed in the reservoirs of cartridges equipped with respective QR codes for identification. The cartridges were installed in the inhaler. The cartridges were properly identified and the inhaler came to a standby state. It was decided that the salbutamol sulfate and then the insulin were ejected in that order. Since the targeted site in the body depends on the medicine, the sizes of droplets, that is, the nozzle diameters of the ejection sections, require to be appropriately selected. The ejection from each cartridge was performed by a thermal ink jet (thermal liquid jet) method, and the nozzle diameters were 7 µm for the salbutamol sulfate liquid and 3 µm for the insulin liquid. The time interval between the ejections of the two ejection liquids from the ejection sections was set at 1.5 seconds. The inhaler was started by pressing the power button 9 shown in Fig. 3. On stopping the ejection of the salbutamol sulfate solution, the ejection of the insulin solution was started, and thus the two solutions were ejected separately one after the other.

The particle size distributions and the time interval of the ejection liquids were measured with a particle size distribution meter (Spray Tech, manufactured by Malvern). As a result, the salbutamol droplets had a mean particle size of 7 µm and the insulin droplets had a mean particle size of 3.1 µm. The operational time interval was coincident with the set interval, that is, 1.5 seconds. The particle size distribution was represented by a span value and it was as small as 0.6 in each ejection. The ejected liquids were collected and their concentrations were measured with a high-performance liquid chromatograph (LC-2000, manufactured by JASCO Corporation). As a result, the concentrations were coincident with the initially set concentrations, that is, 0.5% and 0.4%.

Thus, it has been found that desired amounts of medicines can be ejected one after another with desired droplet diameters at a desired time interval by a single action. Table 1 shows the experimental conditions, measured concentrations, mean particle size, and measured time interval.

### EXAMPLES 2 to 36

Evaluations were performed in the same manner as in Example 1 except that the pharmaceutical compounds, the solutions, the set concentrations, and the set time interval were changed as shown in Table 1. In Table 1, the nozzle diameter was set at 7 µm for pharmaceutical compounds other than DPP-4 inhibitor used in medicine A and 3 µm for DPP-4 inhibitor. All the nozzle diameters for medicine B were set at 3 µm. In these examples, DPP-4 inhibitor is sitagliptin.

As shown in Table 1 showing the mean particle size of droplets, the measured concentration, and the measured time interval, it has been found that desired amounts of medicines can be ejected one after another with a desired droplet diameter at a desired time interval by a single action as in Example 1 even if the types of pharmaceutical compounds and the time interval were changed.

### COMPARATIVE EXAMPLE 1

Evaluation was performed in the same manner as in Example 1 except that the salbutamol sulfate solution and the insulin solution were simultaneously ejected from an ejection apparatus not having a decision section or equivalent. The results are shown in Table 1.

While the measured concentration was coincident with the desired set value, the particle size distribution was not the desired value.

### EXAMPLE 37

In Example 37, two medicine liquids were alternately ejected every millisecond. Menthol and insulin were used as pharmaceutical compounds and were dissolved or dispersed in solutions A and B to prepare 0.5% and 0.4% ejection liquids, respectively. The diameters of both nozzles were set at 3 µm. The order at which the two ejection liquids were ejected from the respective ejection heads was decided by manual operation of the user. The alternate ejection pattern was set at 1,000 cycles. The inhaler was started by pressing the power button 9 shown in Fig. 3 and ejection was performed for 1 second at a frequency of 20 kHz with a cascade impactor connected. The particle size distribution was determined from the amount of medicine remaining in a sieve. Air was supplied at a rate of 28.3 L/minutes. The measured particle size distribution was coincident with the initially set value.

The ejected liquids were collected and their concentrations were measured with a high-performance liquid chromatograph (LC-2000, manufactured by JASCO Corporation). As a result, the concentrations were coincident with the initially set concentrations, that is, 0.5% and 0.4%.

### EXAMPLES 38 to 48

Evaluations were performed in the same manner as in Example 37, except that the pharmaceutical compounds, the solutions, the set concentrations, and the number of ejection cycles were changed as shown in Table 2. All the nozzle diameters for medicines A and B were set at 3 µm.

As shown in Tale 2 showing the mean particle size of droplets and the measured concentration, it has been found that desired amounts of medicines can be ejected one after another with a desired droplet diameter at a desired time interval by a single action as in Examples 37 even if the types of pharmaceutical compounds and the number of ejection cycles were changed.

### COMPARATIVE EXAMPLE 2

Evaluation was performed in the same manner as in Example 37 except that ejection liquids were simultaneously ejected from an ejection apparatus not having a decision section or equivalent. The results are shown in Table 2.

While the measured concentration was coincident with the desired set value, the particle size distribution was not the desired value. EXAMPLES 49 to 55

In Examples 49 to 55, the first medicine was ejected for a certain period before two medicines were alternately ejected every millisecond. Evaluations were performed in the same manner as in Examples 37 to 40 except that ejection of medicine A was selectively performed a predetermined number of times before millisecond-level alternate ejections, as shown in Table 3.

As shown in Table 3 showing the mean particle size and the measured concentration, it has been found that desired amounts of medicines can be ejected one after another with a desired droplet diameter at a desired time interval by a single action in any Example.

### EXAMPLE 56

The ejection frequencies of medicines A and B were changed from those in Example 37. Evaluation was performed in the same manner as in Example 37 except that the ejection frequency for medicine A was fixed to 10 kHz and the ejection frequency for medicine B was set at 10 kHz at the beginning of ejection and gradually varied so as to be 25 kHz at the completion of operation. The results were similar to those of Example 37. The measured concentrations and amounts of the pharmaceutical compounds were coincident with values calculated from the initially set values.

### EXAMPLE 57

The ejection frequencies of medicines A and B were changed from those in Example 49.

The ejection frequency for medicine A was set at 20 kHz at the beginning of ejection and gradually varied so as to be 10 kHz at the completion of operation. The ejection frequency for medicine B was set at 5 kHz at the beginning of ejection and gradually varied so as to be 25 kHz at the completion of operation. Evaluation was performed under such conditions in the same manner as in Examples 49 and 56. The results were similar to those of Example 49 and the concentrations and amounts of the medicines were coincident with values calculated from the initially set values, as in Example 56.

### EXAMPLE 58

In Example 58, a medicine suppressing the side effect of the first medicine was used as the second medicine after the ejection of the first medicine. Menthol and insulin were alternately ejected under the same conditions as in Example 49, and then medicine A of Example 20, cromoglycic acid, was ejected 3 seconds after the completion of the alternate ejections. As a result, the particle size distribution and the measured concentrations were coincident with the desired set values.

### EXAMPLE 59

Evaluation was performed in the same manner as in Example 1 except that the ejection method was changed from the thermal ink jet method to a piezoelectric ink jet (piezoelectric liquid jet) method using a piezoelectric element as an electromechanical conversion element. The results were similar to those of Example 1.

### EXAMPLE 60

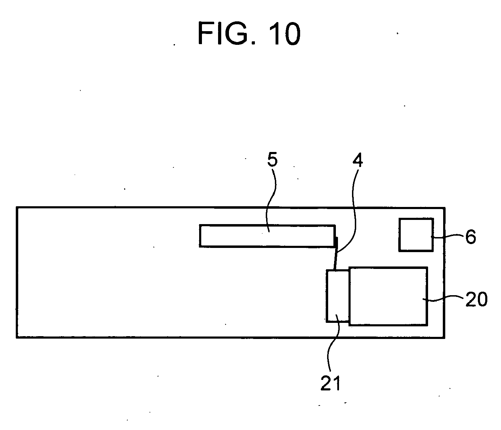

Evaluation was performed in the same manner as in Example 1 except that the ejections of medicines were performed using cartridges shown in Fig. 10 including a mesh type piezoelectric element as a nebulizer. The piezoelectric element had a known structure and was operated under desired conditions based on known information.

### EXAMPLE 61

Evaluation was performed in the same manner as in Example 1 except that salbutamol powder and insulin powder were spray-dried by a known method and classified so that powders had desired particle sizes designated in Example 1. The powders were placed in chambers 20 of cartridges shown in Fig. 10. A plurality of orifices were formed in each chamber and the medicine powders were sprayed with the piezoelectric element 21 through the orifices with the amounts of medicines set at the same as in Example 1. The piezoelectric element had a known structure and was operated under desired conditions based on known information.

Table 4 shows the results of Example 1 and Examples 59 to 61. While the thermal ink jet (thermal liquid jet) and piezoelectric ink jet (piezoelectric liquid jet) methods exhibited very small span values, the nebulizer type and powder type exhibited large span values, that is, wide particle size distributions.

**Table 1**

| | Medicine A | | | | | Medicine B | | | | | Set time interval | Measured time interval |
|---|---|---|---|---|---|---|---|---|---|---|---|---|
| | Main component | Solution | Set concentration | Measured concentration | Particle size µm | Main component | Solution | Set concentration | Measured concentration | Particle size µm | | |
| Example 1 | Salbutamol | A | 0.50% | 0.50% | 7 | Insulin | B | 0.40% | 0.40% | 3.1 | 1.5 | 1.5 |
| Example 2 | Fenoterol | A | 0.50% | 0.50% | 6.9 | Insulin | B | 0.40% | 0.40% | 3 | 1.5 | 1.5 |
| Example 3 | Tiotropium | A | 0.50% | 0.50% | 6.9 | Insulin | B | 0.40% | 0.40% | 3 | 1.5 | 1.5 |
| Example 4 | Ipratropium bromide | A | 0.50% | 0.50% | 7 | Insulin | B | 0.40% | 0.40% | 3 | 1.5 | 1.5 |
| Example 5 | Salbutamol | A | 0.50% | 0.50% | 6.9 | Insulin | B | 0.40% | 0.40% | 3.1 | 3 | 3 |
| Example 6 | Fenoterol | A | 0.50% | 0.50% | 6.9 | Insulin | B | 0.40% | 0.40% | 3 | 3 | 3 |
| Example 7 | Tiotropium | A | 0.50% | 0.50% | 6.9 | Insulin | B | 0.40% | 0.40% | 3 | 3 | 3 |
| Example 8 | Ipratropium bromide | A | 0.50% | 0.50% | 7 | Insulin | B | 0.40% | 0.40% | 3 | 3 | 3 |
| Example 9 | Ipratropium bromide | A | 1.00% | 1.00% | 7 | Insulin | B | 0.40% | 0.40% | 3 | 3 | 3 |
| Example 10 | Ipratropium bromide | A | 1.00% | 1.00% | 7 | Insulin | B | 1.00% | 1.00% | 3 | 3 | 3 |
| Example 11 | Cromoglycic acid | A | 1% | 1% | 7 | Insulin | B | 0.40% | 0.40% | 3.1 | 3 | 3 |
| Example 12 | Acetylcysteine | A | 10% | 10% | 7 | Insulin | B | 0.40% | 0.40% | 3.1 | 3 | 3 |
| Example 13 | Salbutamol | C | 0.50% | 0.50% | 7.1 | Insulin | B | 0.40% | 0.40% | 3.1 | 1.5 | 1.5 |
| Example 14 | Salbutamol | C | 0.50% | 0.50% | 7.1 | Insulin | B | 0.40% | 0.40% | 3.1 | 3 | 3 |
| Example 15 | Fenoterol | C | 0.50% | 0.50% | 6.9 | Insulin | B | 0.40% | 0.40% | 3 | 3 | 3 |
| Example 16 | Tiotropium | C | 0.50% | 0.50% | 6.9 | Insulin | B | 0.40% | 0.40% | 3 | 3 | 3 |
| Example 17 | Ipratropium bromide | C | 0.50% | 0.50% | 7 | Insulin | B | 0.40% | 0.40% | 3 | 3 | 3 |
| Example 18 | Ipratropium bromide | C | 1.00% | 1.00% | 7 | Insulin | B | 0.40% | 0.40% | 3 | 3 | 3 |
| Example 19 | Ipratropium bromide | C | 1.00% | 1.00% | 7 | Insulin | B | 1.00% | 1.00% | 3 | 3 | 3 |
| Example 20 | Cromoglycic acid | C | 1% | 1% | 7 | Insulin | B | 0.40% | 0.40% | 3.1 | 3 | 3 |
| Example 21 | Acetylcysteine | C | 10% | 10% | 7 | Insulin | B | 0.40% | 0.40% | 3.1 | 3 | 3 |
| Example 22 | Ipratropium bromide | A | 0.50% | 0.50% | 7 | Growth hormone | B | 0.50% | 0.50% | 3 | 1.5 | 1.5 |
| Example 23 | Ipratropium bromide | C | 0.50% | 0.50% | 7 | Growth hormone | B | 0.50% | 0.50% | 3 | 1.5 | 1.5 |
| Example 24 | Salbutamol | C | 0.50% | 0.50% | 7 | Growth hormone | B | 0.50% | 0.50% | 3 | 1.5 | 1.5 |
| Example 25 | Fenoterol | C | 0.50% | 0.50% | 7 | Growth hormone | B | 0.50% | 0.50% | 3 | 1.5 | 1.5 |
| Example 26 | Tiotropium | C | 0.50% | 0.50% | 7 | Growth hormone | B | 0.50% | 0.50% | 3 | 1.5 | 1.5 |
| Example 27 | Cromoglycic acid | C | 1% | 1% | 7 | Growth hormone | B | 0.50% | 0.50% | 3 | 1.5 | 1.5 |
| Example 28 | Acetylcysteine | C | 10% | 10% | 7 | Growth hormone | B | 0.50% | 0.50% | 3 | 1.5 | 1.5 |
| Example 29 | Ipratropium bromide | C | 0.50% | 0.50% | 7 | GLP-1 | B | 0.50% | 0.50% | 3 | 1.5 | 1.5 |
| Example 30 | Salbutamol | C | 0.50% | 0.50% | 7 | GLP-1 | B | 0.50% | 0.50% | 3 | 1.5 | 1.5 |
| Example 31 | Fenoterol | C | 0.50% | 0.50% | 7 | GLP-1 | B | 0.50% | 0.50% | 3 | 1.5 | 1.5 |
| Example 32 | Tiotropium | C | 0.50% | 0.50% | 7 | GLP-1 | B | 0.50% | 0.50% | 3 | 1.5 | 1.5 |
| Example 33 | Cromoglycic acid | C | 1% | 1% | 7 | GLP-1 | B | 0.50% | 0.50% | 3 | 1.5 | 1.5 |
| Example 34 | Acetylcysteine | C | 10% | 10% | 7 | GLP-1 | B | 0.50% | 0.50% | 3 | 1.5 | 1.5 |
| Example 35 | Sitagliptin | C | 0.10% | 0.10% | 3.1 | GLP-1 | B | 0.10% | 0.10% | 3 | 1 | 1 |
| Example 36 | Sitagliptin | A | 0.10% | 0.10% | 3.1 | GLP-1 | B | 0.10% | 0.10% | 3 | 1 | 1 |
| Comparative Example 1 | Salbutamol | A | 0.50% | 0.50% | 10 | Insulin | B | 0.40% | 0.40% | 10 | 0 | 0 |

**Table 2**

| | Pattern number of cycles | MedicineA | | | | | Medicine B | | | | |
|---|---|---|---|---|---|---|---|---|---|---|---|
| | | Main component | Solution | Set concentration | Measured concentration | Particle size µm | Main component | Solution | Set concentration | Measured concentration | Particle size µm |
| Example 37 | 1000 | Menthol | A | 0.50% | 0.50% | 3 | Insulin | B | 0.40% | 0.40% | 3 |
| Example 38 | 100 | Menthol | A | 0.50% | 0.50% | 3 | Insulin | B | 0.40% | 0.40% | 3.1 |
| | | | | | | | | | | | |
| Example 39 | 10 | Menthol | A | 0.50% | 0.50% | 3 | Insulin | B | 0.40% | 0.40% | 3.1 |
| Example 40 | 2 | Menthol | A | 0.50% | 0.50% | 3.1 | Insulin | B | 0.40% | 0.40% | 3 |
| | | | | | | | | | | | |
| Example 41 | 1000 | Menthol | C | 0.50% | 0.50% | 3 | Insulin | B | 0.40% | 0.40% | 3 |
| Example 42 | 100 | Menthol | C | 0.50% | 0.50% | 3 | Insulin | B | 0.40% | 0.40% | 3.1 |
| Example 43 | 10 | Menthol | C | 0.50% | 0.50% | 3 | Insulin | B | 0.40% | 0.40% | 3.1 |
| Example 44 | 2 | Menthol | C | 0.50% | 0.50% | 3.1 | Insulin | B | 0.40% | 0.40% | 3 |
| Example 45 | 100 | Fentanyl | A | 1.00% | 1.00% | 3 | Growth hormone | B | 0.50% | 0.50% | 3 |
| Example 46 | 100 | Fentanyl | A | 1.00% | 1.00% | 3 | Insulin | B | 1.00% | 1.00% | 3.1 |
| Example 47 | 1000 | Vanilla essence | A | 0.10% | 0.10% | 3 | Insulin | B | 0.40% | 0.40% | 3 |
| Example 48 | 2 | DHA | A | 0.20% | 0.20% | 3.1 | Growth hormone | B | 0.50% | 0.50% | 3 |
| Comparative Example 2 | 0 | Menthol | A | 0.50% | 0.50% | 7 | Insulin | B | 0.40% | 0.40% | 7 |

**Table 3**

| | Medicine A | | | | | Medicine A ejection number of times | Pattern number of cycles | Medicine B | | | | |
|---|---|---|---|---|---|---|---|---|---|---|---|---|
| | Main component | Solution | Set concentration | Measured concentration | Particle size µm | | | Main component | Solution | Set concentration | Measured concentration | Particle size µm |
| Example 49 | Menthol | C | 0.50% | 0.50% | 3 | 3000 | 1000 | Insulin | B | 0.40% | 0.40% | 3 |
| Example 50 | Menthol | C | 0.50% | 0.50% | 3 | 3000 | 100 | Insulin | B | 0.40% | 0.40% | 3.1 |
| Example 51 | Menthol | C | 0.50% | 0.50% | 3 | 3000 | 10 | Insulin | B | 0.40% | 0.40% | 3.1 |
| Example 52 | Menthol | C | 0.50% | 0.50% | 3.1 | 3000 | 2 | Insulin | B | 0.40% | 0.40% | 3 |
| Example 53 | Menthol | C | 0.50% | 0.50% | 3 | 1000 | 100 | Insulin | B | 0.40% | 0.40% | 3.1 |
| Example 54 | Menthol | C | 0.50% | 0.50% | 3 | 100 | 10 | Insulin | B | 0.40% | 0.40% | 3.1 |
| Example 55 | Menthol | C | 0.50% | 0.50% | 3.1 | 100 | 2 | Insulin | B | 0.40% | 0.40% | 3 |

**Table 4**

| | Medicine A | | Medicine B | |
|---|---|---|---|---|
| | Particle size µm | span | Particle size µm | span |
| Example 1 | 7 | 0.60 | 3.1 | 0.63 |
| Example 59 | 6.9 | 0.60 | 3.1 | 0.63 |
| Example 60 | 7 | 1.8 | 3 | 1.8 |
| Example 61 | 7 | 1.9 | 3 | 2.0 |

While the present invention has been described with reference to exemplary embodiments, it is to be understood that the invention is not limited to the disclosed exemplary embodiments. The scope of the following claims is to be accorded the broadest interpretation so as to encompass all modifications, equivalent structures and functions.

A medicine ejection apparatus ejecting a plurality of medicines contained in a plurality of reservoirs (12, 13) includes a medicine identification section (18a) that identifies the medicines. A decision section (18b) is also provided to decide an ejection order in which the medicines are ejected, according to the combination of the identified medicines. In a medicine ejection section (3), the medicines are ejected in the ejection order decided by the decision section.

## Claims

1. A method for controlling an apparatus for the ejection of at least two medicines, the method comprising the steps of:
setting an ejection order in which the medicines are ejected according to the combination of the medicines, wherein the apparatus comprises a medicine identification section that identifies the medicines; a decision section that sets an ejection order in which the medicines are ejected, according to the combination of the identified medicines; and a memory section that stores information of combinations of a plurality of medicines and ejection orders effective in efficacy for the respective combinations, wherein the decision section collates the combination of the medicines identified by the medicine identification section with the information stored in the memory section and sets the ejection order according to the collation; and
ejecting a first medicine and subsequently ejecting a second medicine according to the ejection order.

2. The method according to Claim 1, wherein the ejection of the second medicine is started after the ejection of the first medicine has been stopped.

3. The method according to Claim 1 or 2, wherein the first medicine is for enhancing an in vivo efficacy of the second medicine.

4. The method according to Claim 1 or 2, wherein the second medicine is for reducing a side effect of the first medicine.

5. The method according to Claim 3, wherein the first medicine has bronchodilating efficacy and the second medicine is intended for transpulmonary absorption or treatment of a deep lung disease.

6. The method according to Claim 5, wherein the medicine intended for transpulmonary absorption contains a diabetic treating agent.

7. The method according to Claim 5, wherein the medicine intended for transpulmonary absorption contains insulin or GLP-1.

8. The method according to Claim 3, wherein the first medicine is a DPP-4 inhibitor and the second medicine is GLP-1.

9. The method according to Claim 3, wherein the first medicine contains an analgesic or a perfume, and the second medicine is intended for transpulmonary absorption or treatment of a deep lung disease.

10. The method according to any one of Claims 1 to 9, wherein the step of ejecting is performed by a liquid jet method.

11. The method according to Claim 10, wherein the liquid jet method ejects liquid by use of thermal energy.

12. A medicine ejection apparatus for ejecting a plurality of medicines contained in a plurality of reservoirs, the apparatus comprising:
a medicine identification section (18a) configured to identify the medicines contained in the reservoirs;
a memory section (19) that stores information of combinations of a plurality of medicines and ejection orders effective in efficacy for the respective combinations,
a decision section (18b) configured to set an ejection order in which the medicines are ejected, according to the combination of the identified medicines; and
a medicine ejection section (3) configured to eject the medicines in the ejection order set by the decision section (18b),
wherein the decision section (18b) is configured to collate the combination of the medicines identified by the medicine identification section with the information stored in the memory section and to set the ejection order according to the collation.

13. The medicine ejection apparatus according to Claim 12, further comprising codes (6) attached to the reservoirs for identifying the medicines, and a code reader (16,17) configured to read the codes to obtain information, wherein the medicine identification section is configured to identify the medicines according to the information obtained by the code reader.

14. The medicine ejection apparatus according to Claim 12 or 13, wherein the medicine ejection section includes an electro-thermal conversion element applying thermal energy to the medicines or an electromechanical conversion element applying mechanical energy to the medicines.

## Patentansprüche

1. Verfahren zum Steuern einer Vorrichtung zur Ausstoßung von zumindest zwei Arzneimitteln, wobei das Verfahren die Schritte umfasst:
Festlegen einer Ausstoßreihenfolge, in welcher die Arzneimittel gemäß der Kombination der Arzneimittel ausgestoßen werden, wobei die Vorrichtung umfasst einen Arzneimittelidentifikationsabschnitt, der die Arzneimittel identifiziert; einen Entscheidungsabschnitt, der eine Ausstoßreihenfolge, in welcher die Arzneimittel ausgestoßen werden, gemäß der Kombination der identifizierten Arzneimittel festlegt; und einen Speicherabschnitt, der Informationen von Kombinationen einer Mehrzahl von Arzneimitteln und Ausstoßreihenfolgen, die effektiv in der Wirksamkeit für die entsprechenden Kombinationen sind, speichert, wobei der Entscheidungsabschnitt die Kombination der Arzneimittel, die durch den Arzneimittelidentifikationsabschnitt identifiziert werden, mit den Informationen, die in dem Speicherabschnitt gespeichert werden, kollationiert und die Ausstoßreihenfolge gemäß der Kollation festlegt; und
Ausstoßen eines ersten Arzneimittels und anschließend Ausstoßen eines zweiten Arzneimittels gemäß der Ausstoßreihenfolge.

2. Verfahren nach Anspruch 1, wobei das Ausstoßen des zweiten Arzneimittels gestartet wird, nachdem das Ausstoßen des ersten Arzneimittels gestoppt wurde.

3. Verfahren nach Anspruch 1 oder 2, wobei das erste Arzneimittel zum Erhöhen einer in vivo Wirksamkeit des zweiten Arzneimittels ist.

4. Verfahren nach Anspruch 1 oder 2, wobei das zweite Arzneimittel für die Reduzierung einer Nebenwirkung des ersten Arzneimittels ist.

5. Verfahren nach Anspruch 3, wobei das erste Arzneimittel eine Bronchodilationswirksamkeit aufweist und das zweite Arzneimittel für transpulmonale Absorption oder die Behandlung einer tiefen Lungenkrankheit vorgesehen ist.

6. Verfahren nach Anspruch 5, wobei das Arzneimittel, das für transpulmonale Absorption vorgesehen ist, ein diabetisches Behandlungsmittel enthält.

7. Verfahren nach Anspruch 5, wobei das Arzneimittel, das für transpulmonale Absorption vorgesehen ist, Insulin oder GLP-1 enthält.

8. Verfahren nach Anspruch 3, wobei das erste Arzneimittel ein DPP-4-Inhibitor ist und das zweite Arzneimittel GLP-1 ist.

9. Verfahren nach Anspruch 3, wobei das erste Arzneimittel ein Analgetikum oder ein Parfüm enthält, und das zweite Arzneimittel für transpulmonale Absorption oder die Behandlung einer tiefen Lungenkrankheit vorgesehen ist.

10. Verfahren nach einem der Ansprüche 1 bis 9, wobei der Schritt des Ausstoßens durch ein Flüssigkeitsstrahlverfahren ausgeführt wird.

11. Verfahren nach Anspruch 10, wobei das Flüssigkeitsstrahlverfahren Flüssigkeit unter Verwendung thermischer Energie ausstößt.

12. Arzneimittelausstoßvorrichtung zum Ausstoßen einer Mehrzahl von Arzneimitteln, die in einer Mehrzahl von Reservoirs enthalten ist, wobei die Vorrichtung umfasst:
einen Arzneimittelidentifikationsabschnitt (18a), der dazu konfiguriert ist, die in den Reservoirs enthaltenen Arzneimittel zu identifizieren;
einen Speicherabschnitt (19), der Informationen von Kombinationen einer Mehrzahl von Arzneimitteln und Ausstoßreihenfolgen, die effektiv in der Wirksamkeit für die entsprechenden Kombinationen sind, speichert,
einen Entscheidungsabschnitt (18b), der dazu konfiguriert ist, eine Ausstoßreihenfolge, in welcher die Arzneimittel ausgestoßen werden, gemäß der Kombination der identifizierten Arzneimittel festzulegen; und
einen Arzneimittelausstoßabschnitt (3), der dazu konfiguriert ist, die Arzneimittel in der durch den Entscheidungsabschnitt (18b) festgelegten Ausstoßreihenfolge auszustoßen,
wobei der Entscheidungsabschnitt (18b) dazu konfiguriert ist, die Kombination der Arzneimittel, die durch den Arzneimittelidentifikationsabschnitt identifiziert sind, mit den in dem Speicherabschnitt gespeicherten Informationen zu kollationieren, und die Ausstoßreihenfolge gemäß der Kollation festzulegen.

13. Arzneimittelausstoßvorrichtung nach Anspruch 12, ferner umfassend Codes (6), die an die Reservoirs angebracht sind, um die Arzneimittel zu identifizieren, und einen Codeleser (16, 17), der dazu konfiguriert ist, die Codes zu lesen, um Informationen zu erhalten, wobei der Arzneimittelidentifikationsabschnitt dazu konfiguriert ist, die Arzneimittel gemäß der Informationen, die durch den Codeleser erhalten ist, zu identifizieren.

14. Arzneimittelausstoßvorrichtung nach Anspruch 12 oder 13, wobei der Arzneimittelausstoßabschnitt ein elektrothermisches Konversionselement, das thermische Energie auf die Arzneimittel anwendet, oder ein elektromechanisches Konversionselement, das mechanische Energie auf die Arzneimittel anwendet, beinhaltet.

## Revendications

1. Procédé pour commander un appareil pour l'éjection d'au moins deux médicaments, le procédé comprenant les étapes qui consistent à :
établir un ordre d'éjection dans lequel les médicaments sont éjectés selon la combinaison des médicaments, où l'appareil comprend une section d'identification de médicaments qui identifie les médicaments ; une section de décision qui établit un ordre d'éjection dans lequel les médicaments sont éjectés, selon la combinaison des médicaments identifiés ; et une section de mémoire qui stocke une information de combinaisons de plusieurs médicaments et des ordres d'éjection efficaces pour les combinaisons respectives, où la section de décision rassemble la combinaison des médicaments identifiés par la section d'identification de médicaments avec l'information stockée dans la section de mémoire et établit l'ordre d'éjection suivant le rassemblement ; et
éjecter un premier médicament et éjecter par la suite un deuxième médicament suivant l'ordre d'éjection.

2. Procédé selon la revendication 1, dans lequel l'éjection du deuxième médicament commence après l'arrêt de l'éjection du premier médicament.

3. Procédé selon la revendication 1 ou 2, dans lequel le premier médicament est destiné à améliorer l'efficacité in vivo du deuxième médicament.

4. Procédé selon la revendication 1 ou 2, dans lequel le deuxième médicament est destiné à réduire un effet secondaire du premier médicament.

5. Procédé selon la revendication 3, dans lequel le premier médicament est efficace en termes de broncho-dilatation et le deuxième médicament est destiné au traitement ou à l'absorption transpulmonaire d'une maladie pulmonaire grave.

6. Procédé selon la revendication 5, dans lequel le médicament destiné à l'absorption transpulmonaire contient un agent de traitement du diabète.

7. Procédé selon la revendication 5, dans lequel le médicament destiné à l'absorption transpulmonaire contient de l'insuline ou un GLP-1.

8. Procédé selon la revendication 3, dans lequel le premier médicament est un inhibiteur de la DPP-4 et le deuxième médicament est un GLP-1.

9. Procédé selon la revendication 3, dans lequel le premier médicament contient un analgésique ou un parfum, et le deuxième médicament est destiné au traitement ou à l'absorption transpulmonaire d'une maladie pulmonaire grave.

10. Procédé selon l'une quelconque des revendications 1 à 9, dans lequel l'étape d'éjection est effectuée par un procédé de jet liquide.

11. Procédé selon la revendication 10, dans lequel le procédé de jet liquide éjecte un liquide en utilisant une énergie thermique.

12. Appareil d'éjection de médicament destiné à éjecter plusieurs médicaments contenus dans une pluralité de récipients, l'appareil comprenant :
une section (18a) d'identification de médicaments configurée pour identifier les médicaments contenus dans les récipients ;
une section de mémoire (19) qui stocke une information de combinaisons d'une pluralité de médicaments et des ordres d'éjection efficaces pour les combinaisons respectives,
une section de décision (18b) configurée pour établir un ordre d'éjection dans lequel les médicaments sont éjectés, selon la combinaison des médicaments identifiés ; et
une section (3) d'éjection de médicaments configurée pour éjecter les médicaments dans l'ordre d'éjection établi par la section de décision (18b),
où la section de décision (18b) est configurée pour rassembler la combinaison des médicaments identifiés par la section d'identification de médicaments avec l'information stockée dans la section de mémoire et pour établir l'ordre d'éjection suivant le rassemblement.

13. Appareil d'éjection de médicaments selon la revendication 12, comprenant en outre des codes (6) fixés aux récipients pour identifier les médicaments, et un lecteur de codes (16, 17) configuré pour lire les codes afin d'obtenir une information, où la section d'identification de médicaments est configurée pour identifier les médicaments suivant l'information obtenue par le lecteur de codes.

14. Appareil d'éjection de médicaments selon la revendication 12 ou 13, dans lequel la section d'éjection de médicaments comporte un élément de conversion électrothermique appliquant une énergie thermique aux médicaments ou un élément de conversion électromécanique appliquant une énergie mécanique aux médicaments.
